# EUROPEAN PATENT APPLICATION

(11) **EP 1 484 319 A1**
(43) Date of publication of application: **08.12.2004**
(21) Application number: 03705267.7
(22) Date of filing: 18.02.2003
(51) Int. Cl.: C07D 213/70, C07D 233/84, A61K 31/4164, A61K 31/4402, A61K 31/695, A61P 3/04, A61P 3/10, A61P 35/00

(54) **CHAIN OLIGOLACTIC ACID THIOESTER**

(30) Priority: 19.02.2002 JP 2002042008
(71) Applicant: AMATO PHARMACEUTICAL PRODUCTS, LTD., Fukuchiyama-shi, Kyoto 620-0932 (JP)
(72) Inventor: WATANABE, Mikio, Hadano-shi, Kanagawa 257-0002 (JP); MURAKAMI, Masahiro, Osaka-shi, Osaka 547-0026 (JP)
(74) Representative: Sternagel, Fleischer, Godemeyer & Partner
(86) International application number: PCT/JP2003/001696
(87) International publication number: WO 2003/076404

(57) **Abstract**

An object of the present invention is to produce and isolate a linear oligolactic acid thioester having a thioester group at the terminus as a single compound. According to the present invention, there is provided a compound represented by the formula (1) or a salt thereof: 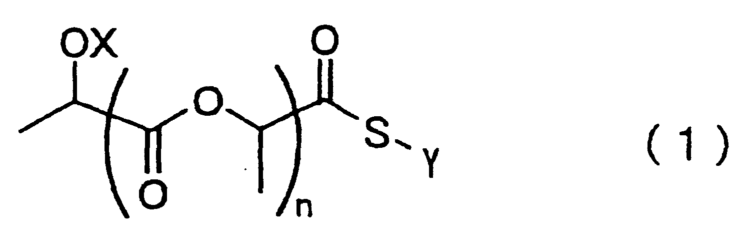 wherein X represents a hydrogen atom or a protective group for a hydroxyl group; Y represents an aliphatic group, an aryl group or a heterocyclic group; and n represents an integer of 0 to 19.

## Description

### TECHNICAL FIELD

The present invention relates to a linear oligolactic acid thioester, and a method for producing the same. Particularly, the present invention relates to a linear oligolactic acid thioester having a thioester group at the terminus, and a method for producing the same.

### BACKGROUND ART

A mixture of cyclic and/or linear poly L-lactic acids having condensation degrees of 3-19 is reported to be useful as an anti-malignant tumor agent (Japanese Patent Laid-open Publication Nos. 9-227388 and 10-130153) and a QOL improving agent for a cancer patient (JP Application No. 11-39894; Journal of Japan Society of Clinical Oncology, vol. 33, No. 3, p493). Furthermore, a mixture of cyclic and/or linear poly L-lactic acids having condensation degree of 3-19 is known to have a blood sugar reducing action so that it is useful as a medicament for prevention and/or treatment of diabetes or a diabetic complication (JP Application No. 11-224883), and is further proved to be useful for suppressing over-appetite, promoting basal metabolism, and improving and/or preventing obesity.

As described above, the mixture of cyclic and/or linear poly L-lactic acids having condensation degree of 3-19 has been demonstrated to exhibit various pharmacological effects, and is expected to be developed as a medicament in future. In order to develop an oligolactic acid as a medicament, it is desirable to isolate it as a single compound having a specific chain length instead of a mixture consisting of poly lactic acids having respective different chain lengths. In order to isolate a polylactic acid having a specific chain length as a single compound, an approach to synthesize a linear oligolactic acid ester having a condensation degree of 3 to 20, of which the terminus carboxyl group has been esterified, has been carried out (JP Application No. 2001-69766). However, there has not yet been any report on synthesis of a compound having a thioester group at the terminus of an oligolactic acid ester.

### DISCLOSURE OF THE INVENTION

A problem to be solved by the present invention is to produce and isolate a linear oligolactic acid thioester having a thioester group at the terminus as a single compound. Another problem to be solved by the invention is to provide a method for producing said compound.

The present inventors made diligent studies to solve the above problems, and as a result, they have succeeded in synthesizing a linear oligolactic acid thioester having a thioester group at the terminus by reacting lactic acid used as a starting material with a disulfide compound. The invention has been completed based on the finding.

Namely, the present invention provides a compound represented by the formula (1) or a salt thereof: 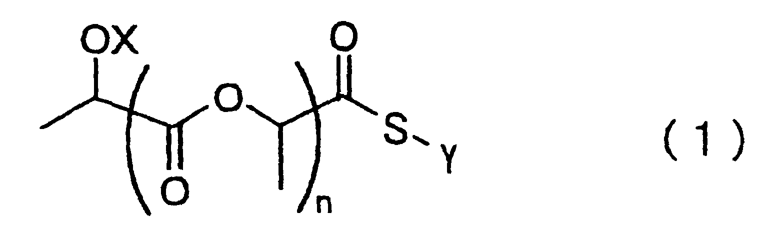 wherein X represents a hydrogen atom or a protective group for a hydroxyl group; Y represents an aliphatic group, an aryl group or a heterocyclic group; and n represents an integer of 0 to 19.

Preferably, n represents an integer of 0 to 5, and particularly preferably, n is 0 or 2.

Preferably, Y is a heterocyclic group, more preferably, Y is a 5-10 membered aromatic heterocyclic group including one or two heteroatoms, and particularly preferably, Y is a pyridyl group or a (4-t-butyl-N-isopropyl)imidazolyl group.

Another aspect of the present invention provides a method for producing a compound represented by the formula: CH₃CH(OX)CO-S-Y, which comprises reacting a compound represented by the formula: CH₃CH(OX)COOH wherein X represents a hydrogen atom or a protective group for a hydroxyl group, with a compound represented by the formula: Y-S-S-Y wherein Y represents an aliphatic group, an aryl group or a heterocyclic group.

The other aspect of the invention provides a method for producing a compound represented by the formula:

CH₃CH(OX)COOCH(CH₃)COOCH(CH₃)CO-S-Y

wherein X represents a hydrogen atom or a protective group for a hydroxyl group, and Y represents an aliphatic group, an aryl group or a heterocyclic group, which comprises reacting a compound represented by the formula:

CH₃CH(OX)COOCH(CH₃)COOH

wherein X represents a hydrogen atom or a protective group for a hydroxyl group, with a compound represented by the formula:

CH₃CH(OH)CO-S-Y

wherein Y represents an aliphatic group, an aryl group or a heterocyclic group.

### BEST MODE FOR CARRYING OUT THE INVENTION

Embodiments and practice methods of the present invention will be described in detail below.

The present invention relates to a compound represented by the following formula (1) and a salt thereof: 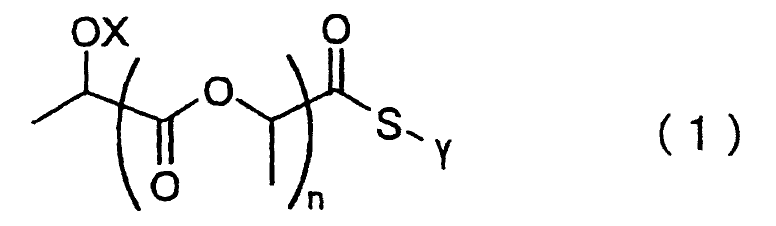 wherein X represents a hydrogen atom or a protective group for a hydroxyl group; Y represents an aliphatic group, an aryl group or a heterocyclic group; and n represents an integer of 0 to 19.

The type of the protective group for a hydroxyl group represented by X is not particularly limited, and may be suitably selected by a person skilled in the art. Specific examples of the protective group for a hydroxyl group include those described below:
(Ether type)
   methyl group, methoxymethyl group, methylthiomethyl group, benzyloxymethyl group, t-butoxymethyl group, 2-methoxyethoxymethyl group, 2,2,2-trichloroethoxymethyl group, bis(2-chloroethoxy)methyl group, 2-(trimethylsilyl)ethoxymethyl group, tetrahydropyranyl group, 3-bromotetrahydropyranyl group, tetrahydrothiopyranyl group, 4-methoxytetrahydropyranyl group, 4-methoxytetrahydrothiopyranyl group, 4-methoxytetrahydrothiopyranyl S,S-dioxide group, tetrahydrofuranyl group, tetrahydrothiofuranyl group;
   1-ethoxyethyl group, 1-methyl-1-methoxyethyl group, 1-(isopropoxy)ethyl group, 2,2,2-trichloroethyl group, 2-(phenylserenyl)ethyl group, t-butyl group, allyl group, cinnamyl group, p-chlorophenyl group, benzyl group, p-methoxybenzyl group, o-nitrobenzyl group, p-nitrobenzyl group, p-halobenzyl group, p-cyanobenzyl group, 3-methyl-2-picolyl N-oxide group, diphenylmethyl group, 5-dibenzosuberyl group, triphenylmethyl group, a-naphthyldiphenylmethyl group, p-methoxyphenyldiphenylmethyl group, p-(p'-bromophenacyloxy)phenyldiphenylmethyl group, 9-anthryl group, 9-(9-phenyl)xanthenyl group, 9-(9-phenyl-10-oxo)anthryl group, and benzisothiazolyl S,S-dioxide group;
   trimethylsilyl group, triethylsilyl group, isopropyldimethylsilyl group, t-butyldimethylsilyl group (TBDMS group), (triphenylmethyl)dimethylsilyl group, t-butyldiphenylsilyl group, methyldiisopropylsilyl group, methyl di-t-butylsilyl group, tribenzylsilyl group, tri-p-xylylsilyl groupl, triisopropylsilyl group, and triphenylsilyl group;
(Ester type)
   formate, benzoyl formate, acetate, chloroacetate, dichloroacetate, trichloroacetate, trifluoroacetate, methoxyacetate, triphenylmethoxyacetate, phenoxyacetate, p-chlorophenoxyacetate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, p-P-phenylacetate, 3-phenylpropionate, 3-benzoylpropionate, isobutylate, monosuccinoate, 4-oxopentanoate, pivaloate, adamantoate, crotonate, 4-methoxycrotonate, (E)-2-methyl-2-butenoate, benzoate, o-(dibromomethyl)benzoate, o-(methoxycarbonyl)benzoate, p-phenylbenzoate, 2,4,6-trimethylbenzoate, p-P-benzoate, and a-naphthoate;
(Carbonate type)
   methylcarbonate, ethylcarbonate, 2,2,2-trichloroethylcarbonate, isobutylcarbonate, vinylcarbonate, allylcarbonate, cinnamylcarbonate, p-nitrophenylcarbonate, benzylcarbonate, p-methoxybenzylcarbonate, 3,4-dimethoxybenzylcarbonate, o-nitrobenzylcarbonate, p-nitrobenzylcarbonate, and S-benzylthiocarbonate; and
(others)
   N-phenylcarbamate, N-imidazolylcarbamate, borate, nitrate, N,N,N',N'-tetramethylphosphorodiamidate, and 2,4-dinitrophenylsulfenate.

A method for introducing and removing a protective group as described above are known to a person skilled in the art, and described in, for example, Teodora, W. Green, Protective Groups in Organic Synthesis, John & Wiley & Sons Inc. (1981) etc.

In the present invention, the type of the aliphatic group, the aryl group or the heterocyclic group represented by Y is not particularly limited.

The aliphatic group in the present invention includes a lower alkyl group, a lower alkenyl group, a lower alkynyl group and the like. The carbon number of the aliphatic group is not particularly limited, but generally 1-10, preferably 1-6, and more preferably 1-4. The chain type of the aliphatic group is not particularly limited, and may be a linear, branched or cyclic chain, or may be any combination of them.

For the aliphatic group in the present invention, a lower alkyl group is particularly preferable. Specific examples of the lower alkyl groups include methyl group, ethyl group, propyl group, isopropyl group, cyclopropyl group, butyl group, isobutyl group, sec-butyl group, tert-butyl group, cyclopropylmethyl group, cyclobutyl group, pentyl group and hexyl group.

For the aryl group in the present invention, an aryl group having a carbon number of 6-24, preferably 6-12 is suitable, and may have one or more substituents. Specific examples of the aryl group include phenyl, naphthyl and p-methoxyphenyl.

For the heterocyclic group in the present invention, a saturated or unsaturated 5-10 membered monocyclic or fused cyclic group containing one or more oxygen atoms, nitrogen atoms or sulfur atoms is suitable. Specific examples of the heterocyclic group include pyridyl, imidazolyl, quinolyl, isoquinolyl, pyrimidinyl, pyrazinyl, pyridazinyl, phthalazinyl, triazinyl, furyl, thienyl, pyrrolyl, oxazolyl, thiazolyl, thiadiazolyl, triazolyl, benzimidazolyl, pyrrolydino and morphorino. The heterocyclic group may have one or more substituents.

Examples of the substituent which may be contained in the aryl group or the heterocyclic group include halogen atom (fluorine, chlorine, bromine or iodine), alkyl group, aryl group, carbonamide group, alkyl sulfonamide group, arylsulfonamide group, alkoxy group, aryloxy group, alkylthio group, arylthio group, carbamoyl group, sulfamoyl group, cyano group, alkylsulfonyl group, arylsulfonyl group, alkoxycarbonyl group, aryloxycarbonyl group and acyl group.

In the invention, Y is preferably aromatic group, and particularly preferably 5-10 membered aromatic heterocyclic group containing one or two hetro atoms. Y is most preferably pyridyl group or (4-t-butyl-N-isopropyl)imidazolyl group.

n represents an integer of 0 to 19. n=0 means monomer of lactic acid, n=1 means dimer of lactic acid, n=3 means trimer of lactic acid, n=3 means tetramer of lactic acid or, similarly, n=19 means eicosamer of lactic acid.

In the present invention, n is preferably an integer of 0 to 5, and in a particularly preferable embodiment, n is 0 or 2.

When X is a hydrogen atom in the formula (1), the compound of the present invention can exist also as a metal salt. Examples of the metal salt include alkali metal salts such as sodium salt and potassium salt, alkali-earth metal salts such as magnesium salt and calcium salt, aluminum salt, and zinc salt.

Various hydrates, solvates and crystal forms of the compound of the present invention are included within the present invention.

Since the compound of the present invention contains an asymmetric carbon, there are stereoisomers. All the possible isomers and a mixture containing two or more types of the isomers at an arbitrary ratio are also included within the present invention. Therefore, the compound of the present invention includes a mixture of various optical isomers such as an optically active substance, a racemic body and a diastereomer, and the isolated single substances thereof.

The configuration of the compound of the present invention depends on that of a lactic acid unit in a compound used as the starting material. Use of the L form, the D form, or a mixture of them as the lactic acid unit in a compound used as a starting material results in compounds of the present invention having various configurations. In the present invention, use of the L form as the configuration of a lactic acid unit is preferred.

Next, the method for producing the compound of the present invention or the salt thereof will be described.

The compound represented by CH₃CH(OX)CO-S-Y, corresponding to a compound of n=0 in the formula (1), can be produced by reacting CH₃CH(OX)COOH wherein X represents a hydrogen atom or a protective group for a hydroxyl group, with a compound represented by the formula Y-S-S-Y wherein Y represents an aliphatic group, an aryl group or a heterocyclic group.

For CH₃CH(OX)COOH used as the starting material, L-lactic acid, D-lactic acid, or a mixture thereof can be employed. A hydroxyl group of the lactic acid may be protected.

When the compound represented by the formula CH₃CH(OX)COOH is reacted with the compound represented by the formula Y-S-S-Y, firstly a triphenylphosphine solution is added to a solution containing the compound represented by the formula CH₃CH(OX)COOH. To the obtained mixture is added a solution containing the compound represented by the formula Y-S-S-Y, and the mixture was stirred for a certain time to allow it to react.

Respective usable amounts of the compound represented by the formula CH₃CH(OX)COOH and the compound represented by the formula Y-S-S-Y may be selected suitably, but the ratio CH₃CH(OX)COOH : Y-S-S-Y is preferably 1:0.5 to 1:2, more preferably 1:0.7 to 1:1.5.

The reaction temperature is not particularly limited as long as the reaction is allowed to proceed, but is preferably -100 °C to room temperature.

The reaction is preferably carried out in the presence of a reaction solvent. The reaction solvent is not particularly limited as long as it is inactive to the reaction, but an aromatic hydrocarbon solvent such as benzene, toluene, xylene and ethylbenzene, as well as tetrahydrofuran (THF), diethyl ether, and dimethoxyethane can be preferably used.

For the reaction atmosphere, an inert gas atmosphere of nitrogen gas, argon gas or the like can be used.

An example of the preferable embodiments of the method of the present invention will be described below.

To a solution of L-lactic acid in toluene is added a solution of triphenylphosphine in toluene under an argon atmosphere at room temperature, and then a solution of a compound represented by the formula Y-S-S-Y (for example, pyridyldisulfide or 2,2-bis-(4-t-butyl-N-isopropyl)imidazolyldisulfide) in toluene is added thereto, followed by stirring for 15 hours. After concentration in a reduced pressure, water is added. The resultant is extracted with chloroform several times, salted out, dried over sodium sulfate, concentrated in a reduced pressure, and isolated and purified by silica gel column chromatography (developing solvent: ether/CHCl₃=1/1) to give L-lactic acid pyridylthiol ester.

The compound represented by CH₃CH(OX)COOCH(CH₃)CO-S-Y, corresponding to a compound of n=1 in the formula (1), can be produced by reacting a compound represented by a formula Y-S-S-Y with CH₃CH(OX)COOCH(CH₃)COOH used as the starting material, in place of CH₃CH(OX)COOH, in the same manner as described above.

The compound represented by the formula CH₃CH(OX)COOCH(CH₃)COOCH(CH₃)CO-S-Y wherein X represents a hydrogen atom or a protective group for a hydroxyl group, and Y represents an aliphatic group, an aryl group or a heterocyclic group, corresponding to a compound of n=2 in the formula (1), can be produced by reacting a compound represented by the formula CH₃CH(OX)COOCH(CH₃)COOH wherein X represents a hydrogen atom or a protective group for a hydroxyl group, with a compound represented by the formula CH₃CH(OH)CO-S-Y wherein Y represents an aliphatic group, an aryl group or a heterocyclic group.

The compound represented by the formula CH₃CH(OH)CO-S-Y can be produced by the method as described above herein.

The compound represented by the formula CH₃CH(OX)COOCH(CH₃)COOH wherein X represents a hydrogen atom or a protective group for a hydroxyl group, is lactoyl lactic acid whose hydroxyl group may be protected if desired. In the present invention, it is preferred to use the lactoyl lactic acid having a protected hydroxyl group (i.e. X is a protective group for a hydroxyl group).

The reaction of a compound represented by the formula CH₃CH(OX)COOCH(CH₃)COOH wherein X represents a protective group for a hydroxyl group, with a compound represented by the formula CH₃CH(OH)CO-S-Y wherein Y is an aliphatic group, an aryl group or a heterocyclic group, is a common esterification reaction.

This esterification reaction can be carried out, for example, by adding a dicyclohexylcarbodimide solution to a solution containing a compound represented by the formula CH₃CH(OX)COOCH(CH₃)COOH, stirring the mixture, adding a solution of a compound represented by the formula CH₃CH(OH)CO-S-Y and a solution of dimethylaminopyridine, and then stirring the mixture for a certain time.

Respective usable amounts of the compound represented by the formula CH₃CH(OX)COOCH(CH₃)COOH and the compound represented by the formula CH₃CH(OH)CO-S-Y may be selected suitably, but the ratio formula CH₃CH(OX)COOCH(CH₃)COOH : formula CH₃CH(OH)CO-S-Y is preferably 1:0.3 to 1:2, and more preferably 1:0.4 to 1:1.5.

The reaction temperature is not particularly limited as long as the reaction is allowed to proceed, but is preferably -100 °C to room temperature.

The reaction is preferably carried out in the presence of a reaction solvent. The reaction solvent is not particularly limited as long as it is inactive to the reaction, but use of toluene, benzene, xylene, alkylbenzene, tetrahydrofuran (THF), diethyl ether, dimethoxyethane or the like may be preferable.

For the reaction atmosphere, an inert gas atmosphere of nitrogen gas, argon gas or the like can be used.

A compound of n=0, 1, or 2 in the formula (1) can be produced in a manner described above. A compound of n=3 also can be synthesized in the same manner as described above by using a thioester compound containing a desired number of lactic acid unit as the starting material, and allowing this compound to be reacted with a compound represented by the formula CH₃CH(OX)COOCH(CH₃)COOH wherein X is a protective group for a hydroxyl group.

The present invention will be described in more detail by the following examples, but the present invention is not limited to the examples.

### EXAMPLES

### Example 1: Synthesis of L-lactic acid pyridylthiol ester

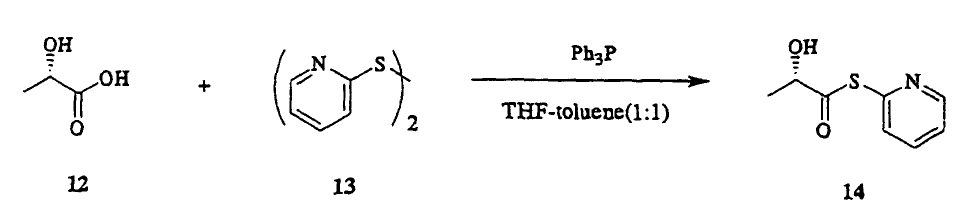

To a solution of L-lactic acid (Compound 12) (0.0901 g (1 mmol)) in toluene (10 ml) was added a solution of triphenylphosphine (0.289 g(1.1 mmol)) in toluene (5 ml) under an argon atmosphere at room temperature, and further, a solution of pyridyldisulfide (Compound 13) (0.242 g (1.1 mmol)) in toluene (5 ml) was added thereto, followed by stirring for 15 hours. After concentration in a reduced pressure, water (30 ml) was added. The resultant was extracted with chloroform (15 ml×3), salted out, dried over sodium sulfate, concentrated in a reduced pressure, and isolated and purified by silica gel column chromatography (developing solvent: ether/CHCl₃=1/1) to give L-lactic acid pyridylthiol ester (Compound 14) (0.0556 g) at a yield of 30%. ([a]²⁰_{D}=-24.55°(C=0.11(CHCl₃))
L-lactic acid pyridylthiol ester (Compound 14)
IR(cm⁻¹): 1737(C=O), 3438(-OH)
¹H-NMR (300MHz, CDCl₃)
- d(ppm)=: 1.59 (3H, d, J=7.2Hz)
4.06 (1H, q, J=7.2Hz)
7.23 (1H, ddd, J=0.9Hz, 5.1Hz, 7.2Hz)
7.41 (1H, d, J=7.8Hz)
7.70 (1H, ddd, J=1.8Hz, 7.5Hz, 8.1Hz)
8.43 (1H, d, J=5.7Hz)

### Example 2: Synthesis of L-lactic acid (4-t-butyl-N-isopropyl)imidazolylthiol ester

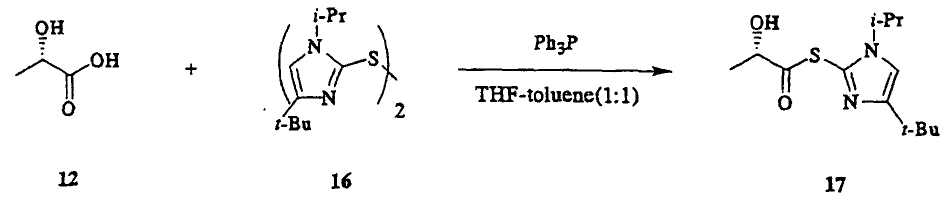

### (Example 2-1)

To a solution of L-lactic acid (Compound 12) (0.450 g (5 mmol)) in a THF-toluene (1:1) mixed solution (50 ml) was added a solution of triphenylphosphine 1.44 g (5.5 mmol) in a THF-toluene (1:1) mixed solution (25 ml) under an argon atmosphere at room temperature, and further a solution of 2,2-bis-(4-t-butyl-N-isopropyl)imidazolyldisulfide (Compound 16) (2.17 g (5.5 mmol)) in a THF-toluene (1:1) mixted solution (25 ml) was added thereto, followed by stirring for 7 hours. After concentration in a reduced pressure, water (30 ml) was added. The resultant was extracted with chloroform (15 mlx3), salted out, dried over sodium sulfate, concentrated in a reduced pressure, and isolated and purified by silica gel column chromatography (developing solvent: ether/CHCl₃=1/1) to give L-lactic acid (4-t-butyl-N-isopropyl)imidazolylthiol ester (Compound 17) (0.308 g) at a yield of 23%. ([a]²⁰_{D}=-80.82°(C=2.0, CHCl₃))
L-lactic acid (4-t-butyl-N-isopropyl)imidazolylthiol ester (Compound 17)
IR(cm⁻¹): 1718(C=O), 3440(-OH)
¹H-NMR (300MHz, CDCl₃)
- d (ppm)=: 1.26 (9H, s)
1.40 (3H, d, J=7.2Hz)
1.45 (3H, d, J=6.6Hz)
1.55 (3H, d, J=7.2Hz)
3.93 (1H, q, J=7.2Hz)
4.34 (1H, sept, J=6.6Hz)
6.65 (1H, s)

### (Example 2-2)

To a solution of L-lactic acid (Compound 12) (0.901 g(10 mmol)) in a THF-toluene (1:1) mixed solution (100 ml) was added a solution of triphenylphosphine (2.89 g (11 mmol)) in a THF-toluene (1:1) mixed solution (50 ml) under an argon atmosphere at 0 °C, and further a solution of 2,2-bis-(4-t-butyl-N-isopropyl)imidazolyldisulfide (Compound 16) (4.34 g (11 mmol)) in a THF-toluene (1:1) mixed solution (50 ml), followed by stirring for 5 hours and further stirring 10 hours at room temperature. After concentration in a reduced pressure, water (30 ml) was added. The resultant was extracted with chloroform (15 mlx3), salted out, dried over sodium sulfate, concentrated in a reduced pressure, and isolated and purified by silica gel column chromatography (developing solvent: ether/CHCl₃=1/1) to give L-lactic acid (4-t-butyl-N-isopropyl)imidazolylthiol ester (Compound 17) 0.463 g at a yield of 17%. The instrumental analysis data of the product coincided with those described above.

### Example 3: Synthesis of O-(t-butyldimethylsilyl)lactoyl lactic acid

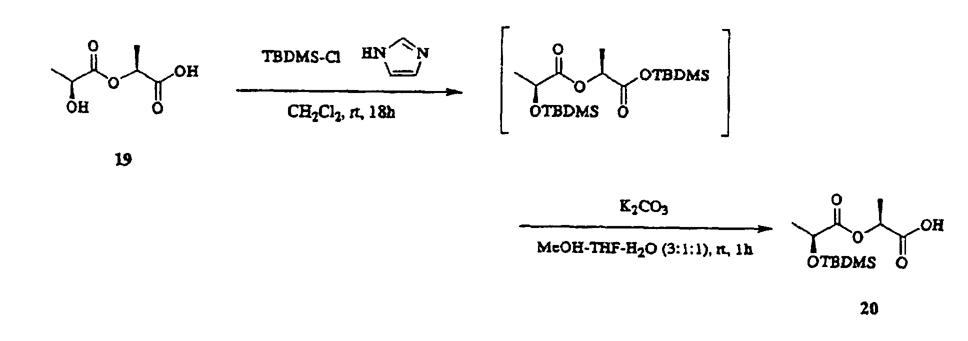

To a solution of imidazole (2.04 g (30 mmol)) in methylene chloride (30 ml) was dropped a solution of t-butyldimethylsilyl chloride (2.06 g (15 mmol)) in methylene chloride (10 ml) under an argon atmosphere at room temperature, followed by stirring for 30 minutes. Then, a solution of lactoyl lactic acid (Compound 19) (0.486 g (3 mmol)) in methylene chloride (10 ml) was added thereto, followed by stirring for 18 hours. Water (200 ml) was added, and the resultant was extracted with ether (200 ml×3), salted out, dried over sodium sulfate, and concentrated in a reduced pressure to give a product 1.15 g. The obtained product was used directly for the following reaction.

At room temperature, to a solution of the obtained product (1.15g) in methanol (30 ml) was added THF (10 ml), and further a solution of potassium carbonate (1.00 g (7.2 mmol)) in water (10 ml) was added thereto, followed by stirring for 1 hour. The solution was concentrated down to a quarter in volume in a reduced pressure, diluted with a saturated saline (30 ml), cooled to 0 °C, and adjusted to a pH of 4-5 with a 1 M potassium hydrogen sulfate solution (12.5 ml). The product was then extracted with ether (50 ml×3), salted out, dried over sodium sulfate, and concentrated in a reduced pressure to give O-(t-butyldimethylsilyl)lactoyl lactic acid (Compound 20) (0.591 g) at a yield of 71%. ([a]²⁰_{D}=-36.91° (C=2.0, CHCl₃)) O-(t-butyldimethylsilyl)lactoyl lactic acid (Compound 20)
IR(cm⁻¹): 1733 (C=O), 3185(-OH)
¹H-NMR (300MHz, CDCl₃)
- d (ppm)=: 0.10 (6H, d, J=7.5Hz)
0.91 (9H, s)
1.45 (3H, d, J=6.6Hz)
1.56 (3H, d, J=7.2Hz)
4.40 (1H, q, J=6.6Hz)
5.12 (1H, q, J=7.2Hz)

### Example 4: Synthesis of trimer (4-t-butyl-N-isopropyl) imidazolylthiol ester

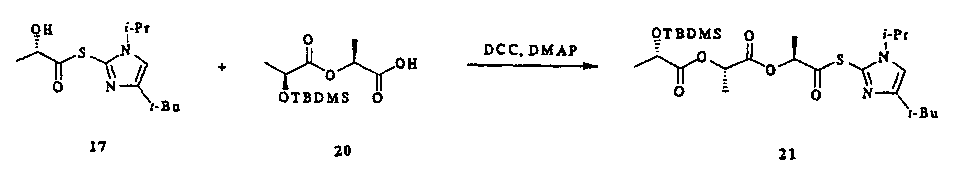

To a solution of O-(t-butyldimethylsilyl)lactoyl lactic acid (Compound 20) (0.276 g (1 mmol)) in toluene (10 ml) was added a solution of dicyclohexylcarbodiimide (0.206 g (1 mmol)) in toluene (5 ml) under an argon atmosphere at room temperature, followed by stirring for 15 minutes. Then, a solution of L-lactic acid (4-t-butyl-N-isopropyl)imidazolylthiol ester (Compound 17) (0.135 g (0.5 mmol)) in toluene (5 ml) and a solution of dimethylaminopyridine (0.0661 g (0.5 mmol)) in toluene (5 ml) were added thereto, followed by stirring for 2 hours. The resultant was treated with a saturated ammonium chloride solution, extracted with ether (30 ml×3), salted out, dried over sodium sulfate, and column-filtered by silica gel column chromatography (ether/hexane=1/1) to give trimer (4-t-butyl-N-isopropyl)imidazolylthiol ester TBDMS product (Compound 21) (0.138 g) at a crude yield of 52%.
¹H-NMR (300MHz, CDCl₃)
- d (ppm)=: 0.09 (6H, d, J=5.1Hz)
0.90 (9H, s)
1.23 (9H, s)
1.29 (3H, d, J=6.6Hz)
1.34 (3H, d, J=7.2Hz)
1.38 (3H, d, J=6.6Hz)
1.42 (3H, d, J=6.9Hz)
1.45 (3H, d, J=6.96Hz)
4.36 (1H, q, J=6.6Hz)
4.56 (1H, sept, J=6.6Hz)
5.25 (1H, q, J=6.9Hz)
5.39 (1H, q, J=6.9Hz)
6.67 (1H, s)

### INDUSTRIAL APPLICABILITY

The present invention makes it possible to provide a linear oligolactic acid thioester having a thioester group at the terminus as a single compound. Use of the single compound of oligolactic acid thioester which is provided by the present invention allows the oligolactic acid thioester to be developed as a drug, a drug raw material, a food additive, a cosmetic raw material, a pharmaceutic raw material and a pharmaceutic additive.

## Claims

1. A compound represented by the formula (1) or a salt thereof: 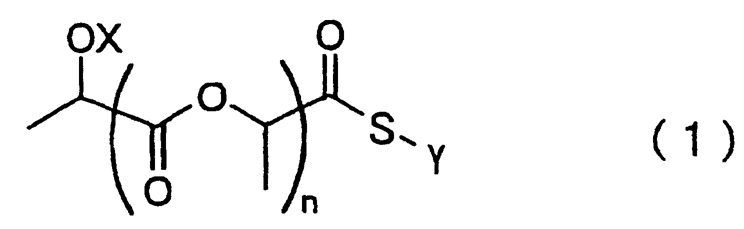 wherein X represents a hydrogen atom or a protective group for a hydroxyl group; Y represents an aliphatic group, an aryl group or a heterocyclic group; and n represents an integer of 0 to 19.

2. The compound according to claim 1 or a salt thereof, wherein n represents an integer of 0 to 5.

3. The compound according to claim 1 or a salt thereof, wherein n is 0 or 2.

4. The compound according to any of claims 1 to 3 or a salt thereof, wherein Y is a heterocyclic group.

5. The compound according to any of claims 1 to 4 or a salt thereof, wherein Y is a 5-10 membered aromatic heterocyclic group including one or two heteroatoms.

6. The compound according to any of claims 1 to 5 or a salt thereof, wherein Y is a pyridyl group or a (4-t-butyl-N-isopropyl)imidazolyl group.

7. A method for producing a compound represented by the formula: CH₃CH(OX)CO-S-Y, which comprises reacting a compound represented by the formula: CH₃CH(OX)COOH wherein X represents a hydrogen atom or a protective group for a hydroxyl group, with a compound represented by the formula: Y-S-S-Y wherein Y represents an aliphatic group, an aryl group or a heterocyclic group.

8. A method for producing a compound represented by the formula:
CH₃CH(OX)COOCH(CH₃)COOCH(CH₃)CO-S-Y
wherein X represents a hydrogen atom or a protective group for a hydroxyl group, and Y represents an aliphatic group, an aryl group or a heterocyclic group, which comprises reacting a compound represented by the formula:
CH₃CH(OX)COOCH(CH₃)COOH
wherein X represents a hydrogen atom or a protective group for a hydroxyl group, with a compound represented by the formula:
CH₃CH(OH)CO-S-Y
wherein Y represents an aliphatic group, an aryl group or a heterocyclic group.
